# EUROPEAN PATENT APPLICATION

(11) **EP 2 818 101 A1**
(43) Date of publication of application: **31.12.2014**
(21) Application number: 14170859.4
(22) Date of filing: 03.06.2014
(51) Int. Cl.: A61B 5/00

(54) **Object information acquiring apparatus and laser apparatus**

(30) Priority: 26.06.2013 JP 2013133904
(71) Applicant: Canon Kabushiki Kaisha, Tokyo 146-8501 (JP)
(72) Inventor: Watanabe, Tadaki, Tokyo,, Tokyo 146-8501 (JP); Nagao, Daisuke, Tokyo,, Tokyo 146-8501 (JP)
(74) Representative: TBK

(57) **Abstract**

Used is an object information acquiring apparatus including an irradiation unit (103) for irradiating an object (101) with a laser beam, a wavelength switching unit (503) for selecting a wavelength of the laser beam, a restriction unit for restricting an output value of the laser beam, a probe (104) for receiving acoustic waves that are generated from the object irradiated with the laser beam, and a configuration unit for generating characteristic information relating to the object in use of the acoustic waves, wherein the restriction unit restricts the output value according to the wavelength selected by the wavelength switching unit.

## Description

### BACKGROUND OF THE INVENTION

### Field of the Invention

The present invention relates to an object information acquiring apparatus and a laser apparatus.

### Description of the Related Art

As one type of light imaging technology using light, there is photoacoustic imaging (PAI). With photoacoustic imaging, a living body as an object is irradiated with pulsed light, and acoustic waves that are generated at an object segment, such as a tumor, based on the energy absorption of the pulsed light are received with a probe. In addition, by subjecting a reception signal output from the probe to analytical processing, optical characteristic distribution in the living body can be acquired as image data.

Japanese Patent Application Publication No. 2010-022812 discloses an apparatus which holds a breast from both sides with holding members, and receives acoustic waves while a probe performs two-dimensional scanning above the holding members. As a result of using a probe to perform two-dimensional scanning, characteristic information relating to a plurality of positions in the object can be acquired.

Moreover, the technique of calculating the abundance ratio of substances with different optical absorption spectrums by using signals of acoustic waves obtained by irradiating light of a plurality of wavelengths from a laser apparatus is being researched.

For example, Journal of Biomedical Optics 14(5), 054007 focuses on the point that the optical absorption spectrums are different with oxygenated hemoglobin and reduced hemoglobin existing in the blood, and describes the method of calculating the oxygen saturation in the blood by using a plurality of wavelengths.

Moreover, Japanese Patent Application Publication No. 2011-229735 describes measuring the wavelength of light output from a laser apparatus, and controlling an output of the laser apparatus based on information relating to the measured wavelength.

With the laser apparatus having a variable wavelength, the gain is different based on the wavelength of the output pulsed light. The gain described above is defined as the ratio relative to the output when a voltage is input. Since the gain is different, even when the same voltage is applied, the output will vary when the wavelength is switched and the laser is irradiated.

Generally speaking, upon applying a photoacoustic wave apparatus to a living body, the irradiated light intensity needs to be restricted to be a predetermined threshold or lower. This value is referred to as a maximum permissible exposure (MPE). In order to achieve the above, for example, there is a method of monitoring the voltage that is input to the pulse light source in the power source circuit, and restricting the voltage that is input upon reaching a certain voltage. According to this method, even if a malfunction occurs, it is possible to prevent an unintended voltage from being input to the pulse light source, and prevent the irradiation of pulsed light exceeding the MPE.

Nevertheless, even though the optical threshold upon monitoring the voltage differs depending on the wavelength depending on the wavelength dependency of the gain, there was not means heretofore for switching the voltage monitoring standard, for each wavelength, in a safety interlock. Thus, it was not possible to restrict the voltage that is input according to the wavelength, and restrict the irradiation of the pulsed light. Consequently, for example, even if the voltage is an optimal output for a certain wavelength, there are cases where the output becomes too great after the wavelength is switched, and there is a possibility that the operation of the laser apparatus is stopped because irradiation is restricted, and, contrarily, a possibility that the irradiation output will become too small.

The present invention was devised in view of the foregoing problems. This invention is developed to restrict laser irradiation according to the selected wavelength in a wavelength variable laser.

Further features of the present invention will become apparent from the following description of exemplary embodiments with reference to the attached drawings.

### SUMMARY OF THE INVENTION

The present invention in its first aspect provides an object information acquiring apparatus as specified in claims 1 to 11.

The present invention in its second aspect provides a laser apparatus as specified in claim 12.

### BRIEF DESCRIPTION OF THE DRAWINGS

FIG. 1 is a block diagram showing the configuration of an object information acquiring apparatus;
FIG. 2 is a conceptual diagram in which irradiation is restricted during wavelength switching;
FIG. 3 is a flowchart showing an example of dropping the voltage during wavelength switching;
FIG. 4 is a conceptual diagram showing the difference in output during wavelength switching; and
FIG. 5 is a schematic diagram showing the main configuration of the laser apparatus.

### DESCRIPTION OF THE EMBODIMENTS

The preferred embodiments of the present invention are now explained with reference to the appended drawings. However, the size, material, shape and relative arrangement of components described below are to be suitably changed depending on the configuration and various conditions of the apparatus to which the present invention is to be applied, and these embodiments are not intended to limit the scope of the present invention to the following descriptions.

In the present invention, acoustic waves include elastic waves referred to as sound waves, ultrasound waves, photoacoustic waves, and photoacoustic ultrasonic waves, and the receiver receives acoustic waves that propagated within the object. In other words, the object information acquiring apparatus of the present invention includes an apparatus that uses the photoacoustic effect of receiving acoustic waves generated in an object by causing the object to be irradiated with light (electromagnetic waves), and acquiring the characteristic information in the object.

The characteristic information in the object acquired in the foregoing case indicates the object information which reflects the initial sound pressure of the acoustic waves that are generated based on light irradiation, the light energy absorption density derived from the initial sound pressure distribution, the absorption coefficient, or the concentration of substances configuring the tissues. The concentration of substances is, for example, the oxygen saturation or oxidized/deoxygenated hemoglobin concentration the like. Moreover, the characteristic information may also be acquired as the distribution information relating to the respective positions in the object rather than as numerical value data. In other words, distribution information such as the absorption coefficient distribution or the oxygen saturation distribution may also be acquired as image data.

The present invention is now explained in detail with reference to the drawings. Note that, as a general rule, the same constituent element is given the same reference numeral and the explanation thereof is omitted. The present invention may also be deemed an operation method or control method of an object information acquiring apparatus or a laser apparatus. The present invention may also be deemed a program for causing an information processing apparatus or the like to implement the control method.

While the details are explained in the respective embodiments, the present invention is characterized in changing the voltage threshold that is subject to irradiation restriction according to the wavelength in a laser apparatus capable of using a plurality of wavelengths.

### <Embodiment 1>

This embodiment explains a method of setting a threshold of the voltage that restricts laser irradiation in the respective wavelengths in an apparatus that uses the photoacoustic effect capable of irradiating light in a plurality of wavelengths.

### (Basic configuration of apparatus)

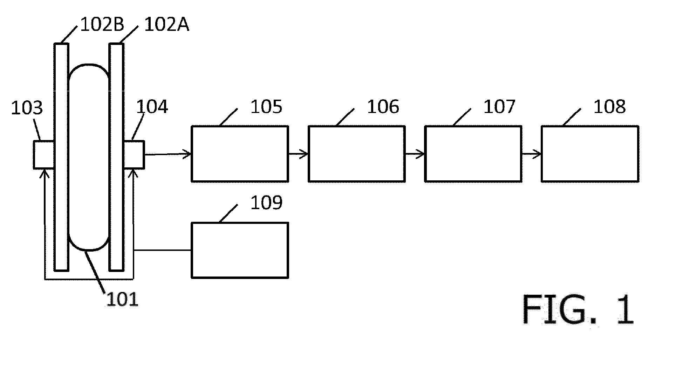

FIG. 1 is a block diagram showing the configuration of the object information acquiring apparatus in this embodiment.

The object information acquiring apparatus comprises holding members 102 for holding an object 101 such as a living body, an irradiation unit 103 for irradiating light, and a probe 104 as a receiver for receiving acoustic waves and converting the received acoustic waves into reception signals. The object information acquiring apparatus further comprises a measuring unit 105 for amplifying the reception signals and converting the amplified reception signals into digital signals, a signal processing unit 106 for performing integration processing and the like of the digitalized reception signals, and an image construction unit 107 for generating image data from output signals from the signal processing unit. The object information acquiring apparatus further comprises an image display unit 108 for displaying an image generated with the image construction unit 107, and a scanning control unit 109 for moving the irradiation unit 103 and the probe 104.

The respective blocks are now explained in detail.

### (Holding members)

As the object 101, considered may be, for example, abreast of a living body. The holding members 102 are configured from a pair of holding members; namely, a first holding member 102A and a second holding member 102B for holding the object 101 from either side. The relative position of both holding members is controlled with a holding mechanism not shown in order to change the holding gap and holding pressure. In the ensuing explanation, when there is no need to differentiate the holding members 102A and 102B, they will be collectively indicated as the holding members 102.

The object 101 is fixed as a result of the holding members 102 sandwiching the object 101, and the measurement error caused by the movement of the object 101 is thereby reduced. Moreover, the object 101 can be adjusted to the intended thickness in accordance with the penetration depth of light. Note that, since the holding member 102B is positioned on the light path of light, it is preferably configured from a material, such as polymethylpentene, with high transmittance relative to the used light. Moreover, the holding member 102A on the side of the probe 104 is preferably configured from a member with high acoustic consistency with the probe 104.

The user opens a door not shown provided to a cabinet and performs procedures for holding the object 101, and thereafter fixes the holding members 102, closes the door, and starts the photography.

### (Irradiation unit)

The irradiation unit 103 for irradiating the object 101 with light is configured from a light source for generating light, and an irradiation part for irradiating the object with light from the light source by guiding light to that object. The irradiation unit corresponds to the irradiation means of the present invention.

As the light source, preferably used is a solid-state laser capable of generating pulsed light (pulse width of 100 nsec or less) having a center wavelength in a near infrared region of 530 to 1300 nm. For example, a Yttrium-Aluminium-Garnet laser or a Titan-Sapphire laser is used. Note that the wavelength of the measuring light is selected between 530 nm and 1300 nm according to the light absorbing substance (for instance, hemoglobin or glucose, or cholesterol) in the object to be measured.

The light source is configured from a pulse-forming network (PFN), a flash lamp, a laser medium, wavelength switching means, a Q-switch, a variable voltage power source, a voltage monitoring circuit, voltage control means, irradiation restriction control means, and voltage threshold switching means.

The voltage control means controls the variable voltage power source. The pulse-forming network accumulates an electrical charge according to the voltage of the variable voltage power source, and generates a high current pulse from the accumulated electric charge. In addition, the high current pulse from the pulse-forming network is sent to the flash lamp and, by consequently exciting the laser medium, a laser beam is emitted. Here, the Q-switch outputs a giant pulse from the excited laser medium.

The voltage monitoring circuit monitors the voltage that is input to the pulse-forming network, and detects the voltage value. In the event the light source is subject to an abnormality and the input voltage increases, when the voltage monitoring circuit detects such voltage increase, the irradiation restriction control means forcibly restricts the irradiation of laser so that the accumulated electric charge does not become excessive, and thereby prevents the laser from being irradiated. The voltage monitoring circuit corresponds to the monitoring means of the present invention.

Various methods may be considered for forcibly restricting laser irradiation. For example, there is the method of suppressing the input of voltage from the variable voltage power source to the pulse-forming network. Moreover, there is the method of suppressing the application of a pulse to the flash lamp. Moreover, there is the method of completely blocking light at the exit end with a shutter in order to prevent irradiation of a laser from the irradiating part described later. Moreover, there is the method of sufficiently weakening the irradiation light with light reduction means such as a filter. Moreover, there is the method of sufficiently weakening the irradiation light per unit area with light diffusion means such as a lens. Moreover, there is the method of not performing Q-switching, which is performed for laser irradiation.

In particular, the method of suppressing the application of voltage from the variable voltage power source to the pulse-forming network can more reliably restrict the irradiation of laser since the laser medium is not excited to begin with.

The light source in this embodiment is a wavelength variable laser capable of irradiating a plurality of wavelengths, and the gain is different depending on the used wavelength. In other words, even when the voltage that is applied to the pulse-forming network is the same, the laser output will differ according to the wavelength. Thus, a phenomenon may occur where the light intensity will not exceed the maximum permissible exposure (MPE) with the wavelength before switching when the voltage is constant, but will exceed the MPE with the wavelength after switching. This kind of phenomenon occurs when the gain is relatively low with the wavelength before switching, and when the gain is relatively high when the wavelength after switching.

Thus, the voltage threshold switching means is used to switch the threshold in the voltage monitoring circuit according to the wavelength so that light exceeding the MPE will not be irradiated even when the used wavelength is switched. This threshold is a numerical value in which the irradiation of light from the irradiation unit is forcibly stopped when the voltage exceeds this value. The threshold according to the wavelength may be obtained in advance for each laser medium or for each individual apparatus, stored in the storage means, and read as needed. Otherwise, the user's input of the threshold may be received from input means not shown. Based on this control, it is possible to prevent a laser beam exceeding the MPE from being irradiated during wavelength switching, and thereby appropriately restrict the laser irradiation.

With a wavelength in which the output increases even when the voltage applied to the pulse-forming network is low; that is, a wavelength in which the output value is relatively large in comparison to the voltage value, the voltage for restricting the laser irradiation is set low. Contrarily, with a wavelength in which the output will not increase unless the voltage applied to the pulse-forming network is increased; that is, a wavelength in which the output value is relatively small in comparison to the voltage value, the voltage for restricting the laser irradiation is set high. It is thereby possible to restrict the laser irradiation by giving consideration to the gain difference.

Prior to selecting the wavelength with the wavelength switching means, the voltage threshold switching means switches the threshold.

A light source normally has a preset irradiation frequency. This is set as a design value for continuously irradiating pulsed light of the intended intensity. Since this irradiation frequency affects the number of times that photoacoustic measurement can be performed per unit time, the higher the irradiation frequency, the better.

As the irradiating part, used may be, for example, a mirror that reflects light, a lens that condenses or expands light or changes the shape thereof, a prism that scatters, bends or reflects light, an optical fiber that propagates light, or a diffuser. The irradiating part may be any kind of component so as long as the intended area of the object is irradiated with light emitted from the light source in an intended shape.

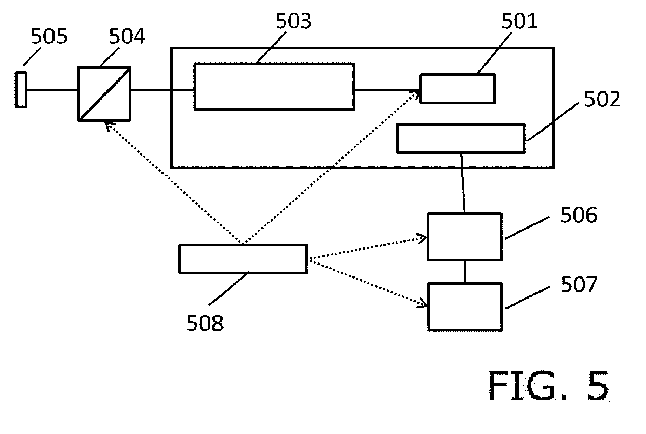

The main constituent elements of the laser apparatus are now explained with reference to FIG. 5. The laser medium 501 is excited with the flash lamp 502, and discharges light via Q-switching. The discharged laser beam is output from the output mirror 505 as irradiation light of an intended wavelength selected by the wavelength switching means 503. Moreover, a shutter 504 may be provided as needed, and output control may be performed. A high current pulse from the pulse-forming network 506 that received a voltage of the variable voltage power source 507 flows into the flash lamp 502.

The control unit 508 controls the foregoing constituent elements. The control unit may be a single control circuit or an information processing apparatus. Otherwise, the control unit may also be a result of the foregoing voltage control means, irradiation restriction control means, voltage threshold switching means and the like collectively comprising the function of restricting the output value of the laser beam. In the foregoing case, the restriction means of the present invention corresponds to the control unit.

The arrow of the dotted line heading from the control unit toward the respective constituent elements indicates the control signal in each of the various methods described above for forcibly restricting the laser irradiation.

### (Probe)

The probe 104 comprises an element for receiving acoustic waves and converting the received acoustic waves into electrical signals (reception signals). As the element of the probe 104, considered may be a conversion element that uses the piezoelectric phenomena, a conversion element that uses the resonance of light, or a conversion element that uses the change in capacitance. Any kind of element so as long as it can receive acoustic waves and convert the received acoustic waves into electrical signals. The use of a probe in which a plurality of elements are disposed one-dimensionally or two-dimensionally is preferable since the measurement can be enlarged, the measurement time can be shortened, and the SN ratio can be improved.

Note that, since the sound pressure of the generated acoustic waves is proportional to the light intensity of light, the area of the front face of the probe is preferably irradiated in order to improve the SN ratio of the reception signals. Thus, the exit end of light of the irradiation unit 103 and the probe 104 are preferably disposed at positions facing each other across the object. Moreover, the scanning control unit 109 preferably performs scanning in synch so as to maintain the positional relationship of the exit end of light and the probe 104. Moreover, as a result of the irradiating part also guiding light to the side of the probe 104, the object 101 can be irradiated with light from the same side as the probe 104.

### (Measuring unit)

The measuring unit 105 is configured from a signal amplification unit for amplifying analog signals (analog reception signals) that are input from the probe 104, and an A/D converter for converting the analog signals into digital signals. The signal amplification unit performs control of increasing or decreasing the amplification gain according to the time from the irradiation of light until the acoustic waves reach the element of the probe in order to obtain image data with an even contrast regardless of the depth in the object.

### (Signal processing unit)

The signal processing unit 106 corrects the sensitivity variation of the element relative to the digital reception signals output from the measuring unit 105, performs compensation processing of a physically or electrically defective element, performs recording operation to a recording medium not shown, performs integration processing for noise reduction, and so on. The integration processing is performed for reducing the system noise by repeatedly receiving acoustic waves at the same scanning position relative to the object 101 and performing averaging processing of the reception signals, and thereby improving the SN ratio of the reception signals.

### (Image construction unit)

The image construction unit 107 uses the signals output from the signal processing unit 106 and acquires, as image data, the distribution (characteristic distribution such as absorption coefficient distribution and oxygen saturation distribution) that indicates the optical characteristic information relating to the respective positions in the object 101. Moreover, various types of correction processing such as brightness adjustment, distortion correction or cutout of attention area may be performed to the generated image data in order to generate image data that is more suitable for diagnosis. The image construction unit corresponds to the construction means of the present invention.

### (Image display unit)

The image display unit 108 receives the input of image data from the image construction unit 107, and displays an image of the characteristic distribution.

### (Scanning control unit)

The scanning control unit 109 controls the scanning position of the exit end of light and the probe 104 as described above. As a result of performing two-dimensional scanning to the object 101 and receiving the acoustic waves at the respective scanning positions, broad characteristic information can be acquired even with a small probe.

While this embodiment adopted a configuration of receiving acoustic waves by performing scanning with the irradiation unit 103 and the probe 104 above the holding member 102, this embodiment can also be applied to an apparatus that manually performs scanning with the probe and performs photoacoustic measuring by using a plurality of wavelengths.

When this embodiment is applied to an apparatus that manually performs scanning with the probe and performs photoacoustic measurement, it is possible to prevent the object from being irradiated with unwanted irradiation light that is not used for the measurement pursuant to the wavelength switching. Particularly when the object is a living body, the irradiation of unwanted light needs to be suppressed.

### <Embodiment 2>

Embodiment 2 is based on the premise that the thresholds of voltages subject to irradiation restriction according to the used wavelength are different. Here, even when the used wavelength is changed, the currently used voltage is also controlled so that irradiation is not restricted.

More specifically, the currently used voltage is controlled to be lower based on the threshold of the voltage for each wavelength, before changing the wavelength, upon changing the used wavelength.

While the configuration of the object information acquiring system of this embodiment is the same as Embodiment 1, the control method of the light source in the irradiation unit 103 differs from Embodiment 1, and this point is explained in detail.

The light source of this embodiment is configured as with Embodiment 1. The laser irradiation method and the method of restricting laser irradiation are also the same as in Embodiment 1.

With the light source in the irradiation unit 103, as described above, the thresholds of voltages subject to irradiation restriction according to the used wavelength are different. Thus, when the wavelength of the laser beam irradiated by the laser apparatus is switched, there are cases where irradiation is restricted.

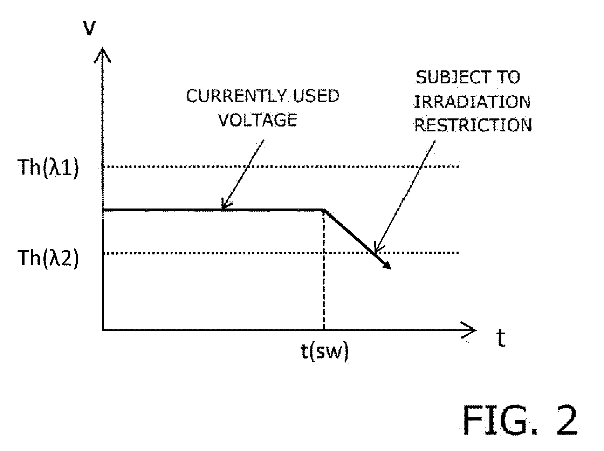

This situation is now explained with reference to FIG. 2. The vertical axis represents the voltage value, and Th(λ1) is the threshold in which the irradiation restriction of the wavelength λ1 will apply, and Th(λ2) is the threshold in which the irradiation restriction of the wavelength λ2 will apply. As shown in the diagram, Th(λ1) > Th(λ2).

The horizontal axis represents the time, and at the timing of t(sw), the wavelength of the laser beam is switched from λ1 to λ2. Consequently, the voltage (currently used voltage) that was usable with the wavelength λ1 since the voltage subject to irradiation restriction was high is subject to irradiation restriction since it becomes a voltage that is subject to irradiation restriction pursuant to the switching to the wavelength λ2.

Various methods may be used for forcibly restricting laser irradiation as described in Embodiment 1. For example, there is the method of suppressing the input of voltage from the variable voltage power source to the pulse-forming network. Moreover, there is the method of suppressing the application of a pulse to the flash lamp. Moreover, there is the method of completely blocking light at the exit end with a shutter in order to prevent irradiation of a laser from the irradiating part described later. Moreover, there is the method of sufficiently weakening the irradiation light with light reduction means such as a filter. Moreover, there is the method of sufficiently weakening the irradiation light per unit area with light diffusion means such as a lens. Moreover, there is the method of not performing Q-switching, which is performed for laser irradiation.

The light source in this embodiment is also a wavelength variable laser capable of irradiating a plurality of wavelengths, and the gain is different depending on the used wavelength. In other words, even when the voltage that is input to the pulse-forming network is the same, the laser output will differ according to the wavelength. Thus, this embodiment is based on the premise that the thresholds of voltages subject to irradiation restriction according to the wavelength as described above are different. By switching the threshold that is used as a reference in the voltage monitoring circuit, the laser irradiation can be appropriately restricted. In other words, the light intensity after the wavelength switching is prevented from exceeding the MPE.

With a wavelength in which the output increases even when the voltage applied to the pulse-forming network is low; that is, a wavelength in which the output value is relatively large in comparison to the voltage value, the voltage for restricting the laser irradiation is set low. Contrarily, with a wavelength in which the output will not increase unless the voltage applied to the pulse-forming network is high; that is, a wavelength in which the output value is relatively small in comparison to the voltage value, the voltage for restricting the laser irradiation is set high. It is thereby possible to restrict the laser irradiation by giving consideration to the gain difference. This kind of threshold setting according to the wavelength is processing that is common with Embodiment 1.

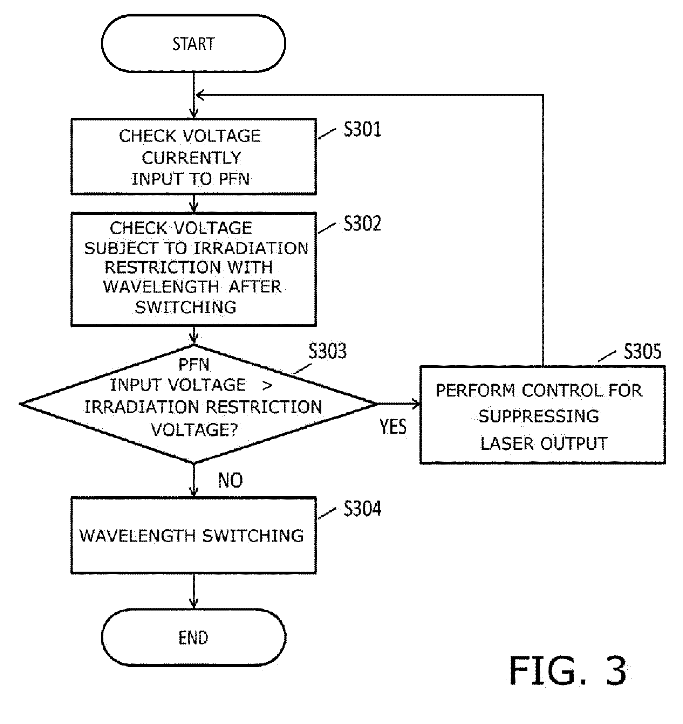

As described above, even when the voltage monitoring circuit monitors the voltage applied to the pulse-forming network in consideration of the gain difference for each wavelength, when the voltage upon switching the used wavelength becomes a value that is subject to irradiation control, the laser irradiation will be restricted due to the switching of the wavelength. Thus, in the light source, the wavelength is switched at the step shown in FIG. 3 upon switching the wavelength in order to prevent the laser irradiation from being restricted.

FIG. 3 is started at the time of switching the wavelength.

Foremost, in step S301, the current voltage that is being input to the pulse-forming network is checked.

Subsequently, in step S302, the voltage subject to irradiation restriction in the wavelength after the wavelength switching is checked.

In step S303, the current voltage that is being input to the pulse-forming network that was checked in S301 and the voltage subject to irradiation restriction that was checked in step S302 are compared.

When the voltage that is being input to the pulse-forming network is lower than the voltage subject to irradiation restriction in the wavelength after the wavelength switching (S303 = NO), the routine proceeds to step S304, and the wavelength is switched.

Meanwhile, when the voltage that is being input to the pulse-forming network is higher than the voltage subject to irradiation restriction in the wavelength after the wavelength switching (S303 = YES), control for lowering the voltage is performed in step S305.

As the method of performing the control for lowering the voltage, there is the method of providing a resistor, and dropping the voltage by applying the voltage that is being input to the pulse-forming network to the resistor. Moreover, there is the method of completely blocking the light at the exit end with a shutter in order to prevent the irradiation of the laser from the irradiating part, then once irradiating the laser, and eliminating all accumulated voltage.

This embodiment described a method of comparing the thresholds of voltages that are subject to irradiation restriction in correspondence with the wavelength, and dropping the voltage as needed. Nevertheless, the voltage can also be dropped regardless of the threshold of the voltage during wavelength switching.

While this embodiment adopted a configuration of receiving acoustic waves by performing scanning with the irradiation unit 103 and the probe 104 above the holding member 102, this embodiment can also be applied to an apparatus that manually performs scanning with the probe and performs photoacoustic measuring by using a plurality of wavelengths.

### <Embodiment 3>

In Embodiment 3, laser irradiation is restricted upon switching the wavelength of the laser in cases of using a plurality of wavelengths of different gain.

While the configuration of the object information acquiring system of this embodiment is the same as Embodiment 1, the control method of the light source in the irradiation unit 103 differs from Embodiment 1, and this point is explained in detail.

The light source of this embodiment is configured, as with Embodiment 1, from a pulse-forming network, a flash lamp, a laser medium, a voltage monitoring circuit and the like. The laser irradiation method and the method of restricting laser irradiation are also the same as in Embodiment 1.

With the light source in the irradiation unit 103, the gain differs with each of the used wavelengths. Accordingly, when the voltage input to the pulse-forming network is caused to be constant, the output will change when the used wavelength is switched. Thus, in the event the light source is subject to an abnormality and the wavelength is switched, if the laser is irradiated unexpectedly, there is a possibility that a strong laser will be irradiated.

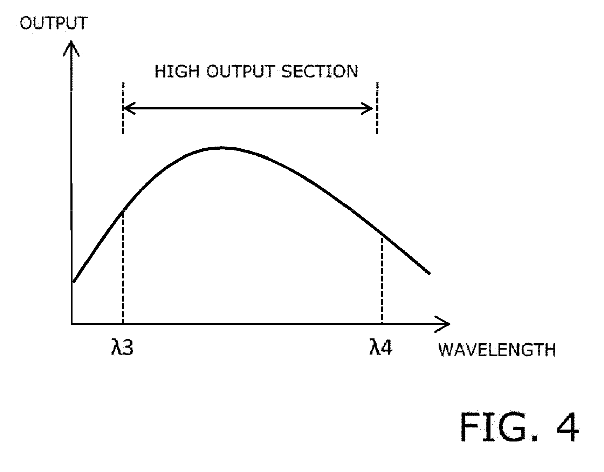

A case of switching from a wavelength λ3 to a wavelength λ4 in a laser apparatus using a certain laser medium is now considered with reference to FIG. 4. The horizontal axis represents the wavelength, and the vertical axis represents the output value of the laser beam corresponding to a certain voltage. Based on the diagram, it can be understood that, when the same voltage is applied to the pulse-forming network, while the output values in the wavelengths λ3 and λ4 are relatively small, the output values in the wavelength region (high output section) between the two wavelengths λ3 and λ4 are relatively large.

Accordingly, if the laser is irradiated due to some sort of abnormality during the switching from the wavelength λ3 to the wavelength λ4, the strength will increase and there is a possibility that it may exceed the MPE.

Thus, in this embodiment, control is performed so as to prevent laser irradiation during the switching of the wavelength. Various control methods may be considered. For example, there is the method of suppressing the input of voltage from the variable voltage power source to the pulse-forming network. Moreover, there is the method of suppressing the application of a pulse to the flash lamp. Moreover, there is the method of completely blocking light at the exit end with a shutter in order to prevent irradiation of a laser from the irradiating part described later. Moreover, there is the method of sufficiently weakening the irradiation light with light reduction means such as a filter. Moreover, there is the method of sufficiently weakening the irradiation light per unit area with light diffusion means such as a lens. Moreover, there is the method of not performing Q-switching, which is performed for laser irradiation.

In particular, the method of suppressing the application of voltage from the variable voltage power source to the pulse-forming network can more reliably restrict the irradiation of laser since the laser medium is not excited to begin with.

While this embodiment adopted a configuration of receiving acoustic waves by performing scanning with the irradiation unit 103 and the probe 104 above the holding member 102, this embodiment can also be applied to an apparatus that manually performs scanning with the probe and performs photoacoustic measuring by using a plurality of wavelengths.

According to the present invention, laser irradiation can be restricted according to the selected wavelength in a wavelength variable laser.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

Used is an object information acquiring apparatus including an irradiation unit for irradiating an object with a laser beam, a wavelength switching unit for selecting a wavelength of the laser beam, a restriction unit for restricting an output value of the laser beam, a probe for receiving acoustic waves that are generated from the object irradiated with the laser beam, and a configuration unit for generating characteristic information relating to the object in use of the acoustic waves, wherein the restriction unit restricts the output value according to the wavelength selected by the wavelength switching unit.

## Claims

1. An object information acquiring apparatus, comprising:
irradiation means configured to irradiate an object with a laser beam;
wavelength switching means configured to select a wavelength of the laser beam;
restriction means configured to restrict an output value of the laser beam;
a probe configured to receive acoustic waves that are generated from the object irradiated with the laser beam;
construction means configured to generate characteristic information relating to the object in use of the acoustic waves; and
a variable voltage power source in which a voltage is controlled by the restriction means,
wherein the restriction means restricts the output value according to the wavelength selected by the wavelength switching means, and
wherein the irradiation means irradiates a laser beam as a result of laser medium excitation by a flash lamp that has received a high current pulse, which was created according to the voltage value of the variable voltage power source in a pulse-forming network, and also a result of Q-switching of a laser medium by a Q-switch.

2. The object information acquiring apparatus according to claim 1, wherein, for each wavelength of the laser beam, the laser medium has a different gain, which represents an output value of the laser beam relative to the voltage value of the variable voltage power source.

3. The object information acquiring apparatus according to claim 2, further comprising:
monitoring means configured to monitor the voltage value that is input from the variable voltage power source to the pulse-forming network,
wherein the restriction means restricts the output value, upon comparing the voltage value detected by the monitoring means and the output value of the laser beam that is obtained from the gain in the wavelength selected by the wavelength switching means.

4. The object information acquiring apparatus according to claim 3, further comprising:
storage means configured to store a threshold that is predetermined for each wavelength of the laser beam with regard to the voltage value,
wherein the restriction means restricts the output value, upon comparing the voltage value detected by the monitoring means and the threshold in the wavelength selected by the wavelength switching means.

5. The object information acquiring apparatus according to claim 4, wherein the threshold is set so that the output value of the laser beam is a value that does not exceed an MPE.

6. The object information acquiring apparatus according to claim 4, wherein, in relation to the threshold in the respective wavelengths, the threshold is set to be lower as the gain is higher.

7. The object information acquiring apparatus according to claim 4, wherein the restriction means restricts the output of the laser beam when the wavelength switching means switches the wavelength.

8. The object information acquiring apparatus according to claim 7, wherein, when the wavelength switching means switches the wavelength of the laser beam from a wavelength in which the threshold is relatively high to a wavelength in which the threshold is low, the restriction means restricts the output value of the laser beam before the wavelength switching means switches the wavelength.

9. The object information acquiring apparatus according to claim 1, wherein the restriction means restricts the output value by suppressing an input of voltage from the variable voltage power source to the pulse-forming network.

10. The object information acquiring apparatus according to any one of claims 2 to 9, wherein the restriction means restricts the output value by suppressing an application of a high current pulse to the flash lamp.

11. The object information acquiring apparatus according to claim 1, wherein the restriction means restricts the output value by preventing the Q-switch from implementing Q-switching.

12. A laser apparatus, comprising:
irradiation means configured to irradiate a laser beam;
wavelength switching means configured to select a wavelength of the laser beam;
restriction means configured to restrict an output value of the laser beam; and
a variable voltage power source in which a voltage is controlled by the restriction means,
wherein the restriction means restricts the output value according to the wavelength selected by the wavelength switching means, and
wherein the irradiation means irradiates a laser beam as a result of laser medium excitation by a flash lamp that has received a high current pulse, which was created according to the voltage value of the variable voltage power source in a pulse-forming network, and also a result of Q-switching of a laser medium by a Q-switch.
